Europäisches Patentamt

European Patent Office

Office européen des brevets

(11) Veröffentlichungsnummer : **0 065 112**
**B1**

(12) **EUROPÄISCHE PATENTSCHRIFT**

(45) Veröffentlichungstag der Patentschrift :
04.07.84

(51) Int. Cl.³ : **C 07 C   1/20, C 07 C   11/02**

(21) Anmeldenummer : **82103253.9**

(22) Anmeldetag : **17.04.82**

(54) Verfahren zur Herstellung von Olefinen.

(30) Priorität : **13.05.81 DE 3118954**

(43) Veröffentlichungstag der Anmeldung :
**24.11.82 Patentblatt 82/47**

(45) Bekanntmachung des Hinweises auf die Patenterteilung : **04.07.84 Patentblatt 84/27**

(84) Benannte Vertragsstaaten :
**BE DE FR GB IT NL**

(56) Entgegenhaltungen :
**EP-A- 0 006 501**
**EP-A- 0 060 103**
**DE-A- 2 928 510**

(73) Patentinhaber : **HOECHST AKTIENGESELLSCHAFT**
**Postfach 80 03 20**
**D-6230 Frankfurt am Main 80 (DE)**

(72) Erfinder : **Vogt, Wilhelm, Dr.**
**Bellerstrasse 74**
**D-5030 Hürth (DE)**
Erfinder : **Glaser, Hermann**
**Magdalenenweg 16**
**D-5042 Erftstadt (DE)**
Erfinder : **Koch, Jürgen, Dr.**
**Am Römerkanal 10a**
**D-5040 Brühl (DE)**

**Beschreibung**

Die Erfindung betrifft ein Verfahren zur Herstellung von $C_2$-bis $C_4$-Olefinen aus Methanol, Dimethylether und gegebenenfalls Wasserdampf enthaltenden Gasgemischen in Gegenwart von Katalysatoren bei Temperaturen von 250 bis 500 °C und Drucken von 0,1 bis 6 bar unter Rückführung der nicht umgesetzten Ausgangsstoffe.

Ein derartiges Verfahren ist im wesentlichen aus der US-PS-4 052 479 bekannt. Die Raumgeschwindigkeiten (liquid hourly space velocity = LHSV) liegen hierbei sehr hoch, nämlich im Bereich von 50-1 000 Liter flüssiges Methanol und Dimethylether je Liter Katalysator und Stunde, um den Umsatz der Ausgangsstoffe auf 5 bis 25 % zu begrenzen.

Ein ähnliches Verfahren, wobei jedoch das Ausgangsgemisch kein Wasser enthält, ist in der US-PS-4 062 905 beschrieben. Die LHSV beträgt dort nur 0,1 bis 200, vorzugsweise 1 bis 20.

Bei beiden Verfahren werden als Katalysatoren verschiedene Arten von Zeolithen verwendet, bevorzugt der Typ H ZSM-5. Die Zeolithe weisen meist ein $SiO_2 : Al_2O_3$-Verhältnis von 10-60 auf und werden mit einem $SiO_2$- oder $Al_2O_3$-Gel als Verfestiger zum Katalysator geformt, der in körniger Form (Korngröße 3-6 mm) in Festbettreaktoren oder als Pulver (Korngröße 30-150 μm) in Wirbelbettreaktoren eingesetzt werden kann. Diese Angaben haben auch für das Verfahren der Erfindung Gültigkeit.

Es ist jedoch bisher noch kein brauchbares Verfahren für die Aufarbeitung der Reaktionsprodukte bekannt geworden. Report No. 111, Process Economics Program, SRI, Menlo Park, California, « Methanol as a chemical raw material » von Park L. Morse, 1977, beschreibt lediglich ein Zweistufen-Verfahren, wobei in einem 1. Reaktor Methanol zu Dimethylether und Wasserdampf und in einem 2. Reaktor Dimethylether seinerseits zu Ethylen und Wasserdampf gespalten wird.

Das Verfahren der Erfindung, beidem man zu Beginn Methanol oder ein Methanol/Wasser-Gemisch im Volumenverhältnis 1 : (0,1 bis 2) verdampft und in einem den Katalysator enthaltenden Reaktor umsetzt, die Reaktionsgase durch Abkühlung teilkondensiert, in einem Abscheider in drei Phasen auftrennt und die aus höheren Aliphaten und Aromaten bestehende Ölphase abzieht, ist man dadurch gekennzeichnet daß man die verbleibende wäßrige Phase und die Gasphase in einer Waschkolonne bei Drucken von 1-40 bar mit Wasser wäscht, über Kopf ein überwiegend $C_2$-bis $C_4$-Olefine enthaltendes gasförmiges Kohlenwasserstoffgemisch abzieht und in an sich bekannter Weise auftrennt, aus der Blase der Waschkolonne eine das nicht umgesetzte Methanol und den als Zwischenprodukt gebildeten Dimethylether enthaltende wäßrige Phase abzieht und daraus in einer Abstreifkolonne Methanol und Dimethylether abdestilliert und in den Reaktor zurückführt, während man aus der Blase der Abstreifkolonne das Wasser abzieht.

Das Verfahren der Erfindung kann weiterhin wahlweise und bevorzugt dadurch gekennzeichnet sein, daß

a) der Katalysator einen Zeolith von Typ H ZSM-5 enthält und die Umsetzung bei Drucken von 0,1-3 bar und, entsprechend dem allmählichen Aktivitätsabfall des Katalysators, bei von 280° bis 360 °C ansteigenden Temperaturen stattfindet ;

b) man durch Steuerung der Reaktionstemperatur das Methanol bei jedem Durchgang jeweils nur bis zu höchstens 80 % zu Kohlenwasserstoffen umsetzt ;

c) die Waschkolonne eine obere Absorptionszone, eine untere Abtriebszone sowie einen Ölabscheider für auskondensierende Kohlenwasserstoffe enthält ;

d) man in der Waschkolonne 1 Liter Waschwasser je 3-200 Normliter über Kopf abgezogenes, gasförmiges Kohlenwasserstoffgemisch einsetzt ;

e) die Temperatur in der Blase um 5-40 °C höher liegt als am Kopf der Waschkolonne ;

f) man einen Teil der aus der Blase der Waschkolonne abgezogenen Waschlösung verdampft und in den Reaktor zurückführt ;

g) die Abstreifzone bei der Siedetemperatur des Wassers betrieben wird ;

h) man von dem aus der Blase der Abstreifkolonne abgezogenen Wasser mindestens einen dem jeweils gebildeten Reaktionswasser entsprechenden Anteil aus dem System ausschleust ;

i) man einen Teil des aus der Blase der Abstreifkolonne abgezogenen Wassers abkühlt und auf den Kopf der Waschkolonne zurückführt ;

j) man einen Teil des aus der Blase der Abstreifkolonne abgezogenen Wassers zur Verdünnung des Methanols in den Reaktor zurückführt.

Die Umsetzung gemäß der Erfindung kann ausgeführt werden, indem man Methanol in dampfförmigem Zustand bei Raumgeschwindigkeiten von LHSV = 0,1-10 l flüssiges Methanol/l Kat. · h über den Katalysator leitet, dessen Temperatur im Bereich von 250 °C bis 500 °C eingestellt wird, und zwar in Abhängigkeit von der Startaktivität des Katalysators und vom im Laufe des Betriebes auftretenden Aktivitätsverlust. Der für einen Katalysator vom H ZSM-5-Typ einzuhaltende engere Temperaturbereich beginnt in frischem Katalysatorzustand bei 280 °C und sollte nach allmählichem Aktivitätsverlust bei 360 °C enden, da eine Kompensation des Aktivitätsverlustes durch Temperaturerhöhung über 360 °C hinaus den unerwünschten Gehalt an höheren (ab $C_5$), insbesondere aromatischen Kohlenwasserstoffen im Reaktionsprodukt-Gemisch steigert. In diesem sind außer aliphatischen Verbindungen Benzol, Toluol und Isomere des Xylols, Tri- und Tetramethylbenzols enthalten.

Dem Ziel dieser Erfindung, einen möglichst hohen Anteil niederer Olefine ($C_2$-$C_4$) als Reaktionspro-

dukt zu erzeugen, wirkt die Steigerung des Reaktionsdruckes entgegen. Es ist zwar prinzipiell möglich, die Methanolspaltung bei Drucken oberhalb 10 bar auszuführen, doch sollte man im Hinblick auf die vorgenannte Absicht einen Reaktordruck von 6 bar, bevorzugt 3 bar, nicht überschreiten.

Ein Zusatz von Wasser zum Methanol im Volumenverhältnis (0,1-2) : 1 drängt die Bildung der höheren Kohlenwasserstoffe zugunsten der gewünschten niederen Olefine zurück.

Unter Einhaltung dieser Voraussetzungen ist der Umsatz von Methanol zu niederen Olefinen zusätzlich in erheblichem Maße vom Methanolumsatz überhaupt abhängig, wie aus der folgenden Tabelle zu ersehen ist :

| Umsatz von $CH_3OH$ zu Kohlenwasserstoffen allgemein in %: | $C_2$ | $C_3$ | Öl |
| --- | --- | --- | --- |
| | (Mol% C vom einges. C) | | |
| 90 | 24,7 | 11,8 | 28,9 |
| 81 | 29,9 | 15,5 | 22,3 |
| 69 | 31,6 | 18,2 | 19,3 |
| 59 | 31,8 | 20,2 | 17,3 |
| 51 | 33,8 | 22,2 | 15,6 |
| 44 | 34,0 | 22,8 | 11,9 |

Es liegt deshalb nahe, sich auf Methanol-Umsätze zu Kohlenwasserstoffen $\leqslant 80\%$ beschränken, wobei ein Teil des nicht zu Kohlenwasserstoffen umgesetzten Methanols bis zur Zwischenstufe Dimethylether reagiert.

Es ist ein wesentlicher Bestandteil des hier beschriebenen Verfahrens, im einfachen Durchgang durch den Reaktor nicht zu Kohlenwasserstoffen umgesetztes Methanol und Dimethylether im Kreislauf zu führen und auf diese Weise vollständig umzusetzen. Diese Bedingung muß von einem technisch brauchbaren Verfahren erfüllt werden können.

Zur Rückführung des nichtumgesetzten Methanols und des Dimethylethers eignet sich eine Wasserwäsche mit angeschlossener Abstreifkolonne, wobei die Waschkolonne unter dem Druck des Reaktors betrieben werden kann ; jedoch ist es wirtschaftlich vorteilhaft, den Betriebsdruck zwecks Verringerung der Waschwassermenge vermittels eines zwischen Reaktor und Waschkolonne angeordneten Kompressors auf erhöhten Druck bis zu 40 bar, vorzugszugsweise bis zu 20 bar, zu bringen, da das erzeugte olefinische Kohlenwasserstoffgas in der nicht mehr zur Erfindung gehörenden nachgeschalteten Trennanlage ohnehin unter erhöhten Druck weiterverarbeitet werden muß.

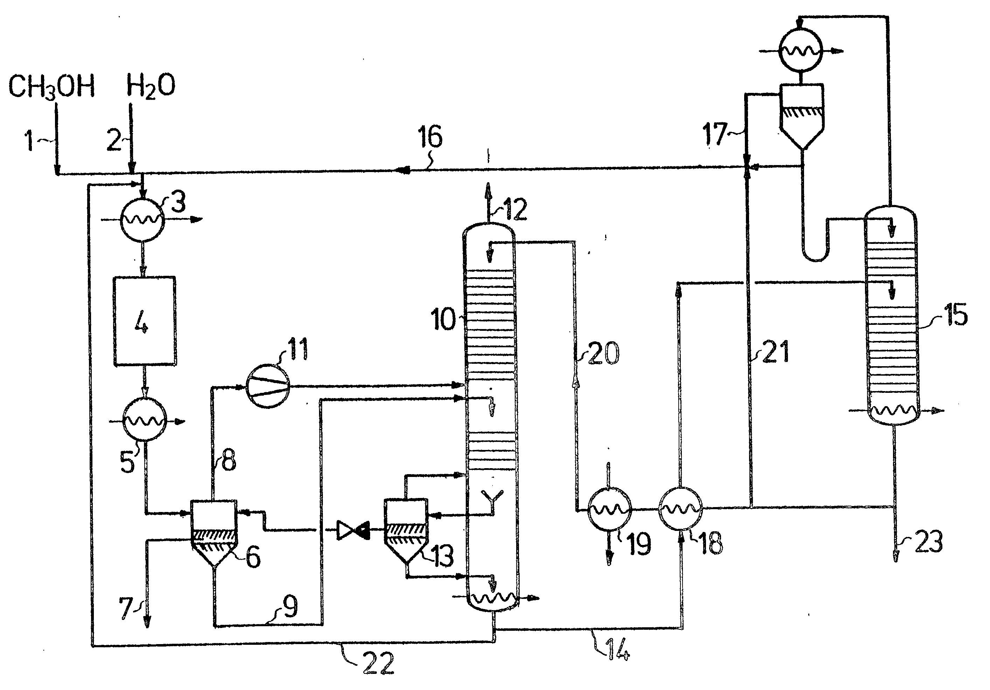

Die erfindungsgemäße Arbeitsweise sei anhand der Zeichnung erläutert, wobei Förderorgane (ausgenommen Kompressor 11) nicht gezeichnet sind.

Über die Leitungen 1 und 2 werden Methanol bzw. Wasser im Verhältnis 1 : (0 bis 2) über den Verdampfer 3 dem Reaktor 4 zugeführt. Der Reaktor 4 ist entweder ein Festbettreaktor, der mit Zeolith-Katalysator gefüllt ist oder ein Wirbelbettreaktor einschließlich Abscheidevorrichtung zur Vermeidung staubförmigen Austrags. Das aus einem breiten Spektrum aliphatischer und aromatischer Kohlenwasserstoffe sowie unumgesetztem Methanol und Dimethylether bestehende gasförmige Reaktionsprodukt wird im Kondensator 5 bei ca. 25 °C teilverflüssigt und im Abscheider 6 in eine Wasser-, eine Öl- und eine Gasphase zerlegt, von denen die aus höheren Aliphaten und Aromaten bestehende Ölphase, die einen guten Kraftstoff (Benzin) darstellt, bei 7 aus der Anlage abgezogen wird, während die Wasser- und Gasphase über die Leitungen 8 bzw. 9 in die Waschkolonne 10 eingeleitet werden. Der Gasstrom kann mit Hilfe des Kompressors 11 auf einen Druck von bis zu 40 bar gebracht werden, unter dem dann auch die Waschkolonne 10 steht, die eine (obere), aus beispielsweise 20 Glockenböden bestehende Absorptionszone sowie eine (untere) Abtriebszone enthält. Der Absorptionsbereich wird mit 1 l Waschwasser von z. B. 20-40 °C je 3-200 l über die Leitung 12 ausströmendem Produktgas (120-200 l bei 40 bar ; 60-100 l bei 20 bar ; 3-5 l bei 1 bar, wobei die Gasliter jeweils unter Normalbedingungen (1,013 bar und 273,15 K) gemessen sind) beaufschlagt, das nach Trocknung einer Tieftemperatur-Gaszerlegung zugeführt wird, die nicht Gegenstand der Erfindung ist.

Das Waschwasser löst Methanol und Dimethylether aus dem Produktgasstrom ; ebenso werden in geringem Umfange Kohlenwasserstoffe gelöst, so daß es zweckmäßig ist, diese in der Abtriebszone der Kolonne 10 durch von unten entgegenströmenden Dimethylether auszutreiben, wobei die Blase der Waschkolonne auf z. B. 30-60 °C geheizt wird. Die Temperatur in der Blase sollte stets 5 bis 40 °C höher liegen als die Kopftemperatur der Waschkolonne 10, damit ein Teil des Dimethylethers ausgetrieben werden und in der Abtriebszone nach oben strömen kann. Infolge der Volumenverminderung der Produktgasmenge beim Lösen des Dimethyletheranteils in Wasser tritt Übersättigung des Gasstromes an höheren Kohlenwasserstoffen ein, so daß diese teilweise als Ölphase in der Waschkolonne ausfallen, über den Ölabscheider 13 ausgeschleust und gegebenenfalls nach Entspannen zwecks Entgasung dem Abscheider 6 zugeführt werden. Die aus der Blase der Waschkolonne 10 abgezogene Waschlösung wird über die Leitung 14 in die Abstreifkolonne 15 eingeleitet und in dieser von Methanol und Dimethylether befreit, die in teils flüssigem, teils gasförmigem Zustand über die Leitungen 16 bzw. 17 in den Reaktor 4 zurückgeleitet werden. Der Betriebsdruck in der Abstreifkolonne 15 ist variabel, so daß der Druck, unter dem Methanol und Dimethylether an deren Kopf abgezogen werden, auf den Reaktoreingangsdruck abgestimmt werden kann.

Das in der Blase der Abstreifkolonne 15 anfallende Wasser (Temperatur z. B. 103-108 °C) wird teilweise nach erforderlicher Kühlung mittels der Wärmeaustauscher 18 und 19 über die Leitung 20 in die Waschkolonne 10 als Waschwasser von z. B. 20-40 °C zurückgeführt und gegebenenfalls teilweise über die Leitung 21 zum Reaktor 4 zurückgeleitet, wobei der Wasserzusatz durch Leitung 2 unterbunden werden kann. Alternativ wird der gleiche Zweck, das für den Reaktor gegebenenfalls benötigte Wasser aus dem Waschkreislauf zu entnehmen, auch dadurch erreicht, daß man die Abstriefkolonne 15 bei hoher Kopftemperatur, bevorzugt oberhalb 90 °C, betreibt, so daß das durch die Leitungen 16 und 17 zum Reaktor 4 zurückfließende Kopfprodukt die gewünschte, für den Reaktor erforderliche stündliche Wassermenge mitbringt. Noch einfacher ist es, statt dessen gleich einen Teil der aus der Blase der Waschkolonne 10 abgezogenen Waschlösung über Leitung 22 in den Verdampfer 3 zurückzuführen. Die bei jedem Durchgang aus Methanol bzw. Dimethylether abgespaltene Wassermenge wird über Leitung 23 abgezogen, so daß die gesamte Wassermenge innerhalb der Anlage konstant bleibt.

Als Vorteil der hier beschriebenen Arbeitsweise ist hervorzuheben, daß dabei nur ein einziger Reaktor erforderlich ist und nicht umgesetztes Methanol und Dimethylether nicht in einem weiteren Reaktor umgesetzt werden müssen.

## Beispiel 1

Über die Eingangsleitungen 1 und 2 werden stündlich 3 l Methanol (= 2 377 g $CH_3OH$) und 3 l Wasser (Vol.-Verhältnis 1 : 1) in den Verdampfer 3 gepumpt. Das verdampfte Methanol-Wasser-Gemisch wird mit einer Anfangstemperatur von 320 °C und bei 1,2 bar Druck in den Reaktionsofen 4 geleitet. Dieser Festbettreaktor 4 ist mit 1 l eines 3 mm Extrudates aus 80 Gew.% H ZSM-5-Zeolith und 20 Gew.% Bindemittel gefüllt. Die LHSV ist demnach 3. Das den Reaktor verlassende Reaktionsgemisch wird im nachgeschalteten Kondensator 5 auf etwa 25 °C abgekühlt und im Abscheider 6 in eine Öl-, eine Wasser- und eine Gasphase zerlegt, von denen die aus höheren Aliphaten und Aromaten bestehende Ölphase über Leitung 7 aus der Anlage abgezogen wird. Die wäßrige Phase wird über die Leitung 9 auf den Abtriebsteil der Waschkolonne 10 gepumpt, während die gasförmigen Produkte über die Leitung 8 mit dem Kompressor 11 bei 20 bar der Waschkolonne 10 unterhalb des Absorptionsteils zugeführt werden. Der Kopf der Waschkolonne 10 wird stündlich über die Leitung 20 mit ca. 5 l Wasser von 25 °C beaufschlagt, um das im Reaktor 4 nicht umgesetzte Methanol und den als Zwischenprodukt auftretenden Dimethylether aus dem Gasgemisch auszuwaschen. Über die Leitung 12 können aus der Waschkolonne 10 stündlich ca. 417 l (gerechnet bei 1,013 bar und 273,15 K) eines Gasgemisches bestehend aus (Vol.%) :

0,4 % CO
3,7 % $CH_4$
51,2 % $C_2H_4$
31,5 % $C_3H_6$
11,8 % $C_4$-Kohlenwasserstoffe
1,4 % höhere Kohlenwasserstoffe

erhalten werden. (Die partialdruckmäßig vorhandenen etwa 2,5 Vol.% Wasserdampf sind hierbei unberücksichtigt gelassen.) Die gesammelten Ölanteile (363 g/h) aus dem Abscheider 6 und dem Ölabscheider 13 werden bei 7 abgezogen. Die Sumpftemperatur im Abtriebsteil der Waschkolonne 10 beträgt 40 °C. Die Temperatur des Reaktors 4 ist mit 320 °C so eingestellt, daß bei einfachem Durchgang 80 % des Methanols zu Kohlenwasserstoffen umgesetzt werden. Nicht umgesetztes Methanol (8 %) und entstandener Dimethylether (12 %, bzw. auf eingesetztes Methanol) werden zusammen mit dem Waschwasser über die Leitung 14 der Abstreifkolonne 15 zugeführt, deren Sumpf auf 104 °C beheizt ist. Im Kopf der Kolonne 15 werden über die Leitung 16 190 g/h Methanol und (über Leitung 17) 205 g/h (100 Normliter/h gasförmiger Dimethylether nach Passieren des Verdampfers 3 in den Reaktor 4 zurückgeleitet.

Unter diesen Bedingungen werden, auf eingesetztes Methanol bezogen, im Durchschnitt an flüssigen und gasförmigen Reaktionsprodukten erhalten (C-%) :

0,1 % CO
1,0 % $CH_4$
26 % Ethylen
24 % Propylen
12 % Butylene
36,9 % höhere aliphatische und aromatische KW.

4

Das Absinken der Aktivität des Katalysators wird durch Anhebung der Temperatur bis 360 °C ausgeglichen. Nach 24 Stunden schaltet man auf einen bereitgehaltenen Reaktor mit reaktiviertem Katalysator um. Gleichzeitig wird der desaktivierte Katalysator oxydativ regeneriert.

Beispiel 2

Man verfährt wie in Beispiel 1, jedoch mit folgenden Ausnahmen :

Die Temperatur im Reaktor 4 wird bei 300 °C gehalten, wodurch der Umsatz des Methanols zu Kohlenwasserstoffen im einfachen Durchgang auf 60 % begrenzt wird. Über die Leitung 16 werden 380 g/h Methanol und (über Leitung 17) 195 g/h gasförmiger Dimethylether in den Reaktor 4 zurückgeleitet. Aus den Abscheidern 6 und 13 wird über Leitung 7 310 g/h Ölphase abgezogen. Über Leitung 12 fallen 458 l/h (gerechnet bei 1,013 bar und 273,15 K) des folgenden Gasgemisches an (Vol.%) :

0,7 % CO
3,1 % $CH_4$
50,7 % $C_2H_4$
31,3 % $C_3H_6$
13,6 % $C_4$-KW

Die partialdruckmäßig vorhandenen 2,5 Vol.% Wasserdampf sind hierbei unberücksichtigt gelassen.

Die Ausbeute an flüssigen und gasförmigen Reaktionsprodukten, bezogen auf das eingesetzte Methanol, beträgt für

| | |
|---|---|
| CO = | 0,2 C-% |
| $CH_4$ = | 1,0 C-% |
| $C_2H_4$ = | 28,0 C-% |
| $C_3H_6$ = | 25,9 C-% |
| $C_4H_8$ = | 13,0 C-% |
| höhere Aliphaten und Aromaten = | 31,5 C-% |

Beispiel 3

Analog den in Beispiel 1 angegebenen Bedingungen werden bei 320 °C über 1 l Katalysator 3 l Methanol (= 2 377 g) und 3 l Wasser geleitet. Die gasförmigen Produkte werden nach Abkühlung auf 25 °C in Kondensator 5 und nach Abscheidung einer Öl- und Wasserphase im Abscheider 6 über die Leitung 9 ohne Kompression der Waschkolonne 10 zugeführt. Auf den Kopf der Kolonne 10 werden stündlich 150 l Wasser von 25 °C zur Entfernung des im Reaktionsgas enthaltenen nicht umgesetzten Methanols und des Dimethylethers gepumpt. Zur Verringerung der im Waschwasser gelösten Kohlenwasserstoffe wird der Sumpf der Kolonne 10 auf 40 °C erwärmt. Am Kopf der Waschkolonne 10 werden stündlich im Mittel 460 Normliter eines Gasgemischs bestehend aus

0,7 Vol.% CO
3,6 Vol.% $CH_4$
47,0 Vol.% $C_2H_4$
28,9 Vol.% $C_3H_6$
10,8 Vol.% $C_4H_8$
9,0 Vol.% höhere KW ($C_5$-$C_8$).

abgezogen. Die partialdruckmäßig vorhandenen etwa 2,5 Vol.% Wasserdampf sind hierbei unberücksichtigt gelassen. Aus den Abscheidern 6 und 13 werden stündlich 214 g Flüssigprodukte abgezogen, die hauptsächlich aus den Aromaten Benzol, Toluol, den Xylolen und Mesitylen bestehen. Über die Leitung 16 werden 190 g/h Methanol und (über Leitung 17) 205 g/h gasförmiger Dimethylether in den Reaktor 4 zurückgeleitet. Aus dem eingesetzten Methanol werden erhalten (C-%) :

0,2 % CO
1,0 % $CH_4$
26,0 % $C_2H_4$
24,0 % $C_3H_6$
12,0 % $C_4H_8$
36,8 % höhere Aliphaten und Aromaten.

Beispiel 4

Analog den in Beispiel 3 angegebenen Bedingungen werden bei 320 °C über 1 l Katalysator 3 l

Methanol (= 2 377 g), jedoch kein Wasser, geleitet. Am Kopf der Waschkolonne 10 werden stündlich im Mittel 415,5 Normliter eines Gasgemisches bestehend aus :

1,2 Vol.% CO
4,0 Vol.% $CH_4$
42,65 Vol.% $C_2H_4$
27,23 Vol.% $C_3H_6$
12,8 Vol.% $C_4H_8$
12,1 Vol.% höhere KW ($C_5$-$C_8$) entnommen.

Aus den Abscheidern 6 und 13 werden stündlich 242 g an Flüssigprodukten abgezogen, die hauptsächlich aus den Aromaten Benzol, Toluol, den Xylolen und Mesitylen bestehen. Über die Leitung 16 werden 170 g/h Methanol und (über Leitung 17) 185 g/h gasförmiger Dimethylether in den Reaktor 4 zurückgeleitet. Aus dem eingesetzten Methanol werden erhalten (C-%) :

0,3 % CO
1,0 % $CH_4$
21,3 % $C_2H_4$
20,4 % $C_3H_6$
12,8 % $C_4H_8$
42,7 % höhere Aliphaten und Aromaten.

Beispiel 5

Man verfährt wie in Beispiel 1. Der Druck in der Waschkolonne 10 wird auf 40 bar erhöht. Über die Leitung 12 werden der Waschkolonne 10 stündlich 396 Normliter eines Gases bestehend aus :

0,42 Vol.% CO
4,2 Vol.% $CH_4$
54,63 Vol.% $C_2H_4$
31,93 Vol.% $C_3H_6$
8,82 Vol.% $C_4$-KW

entnommen. Die bei 7 aus den Ölabscheidern 6 und 13 abgezogenen Flüssigprodukte fallen in einer Menge von 422 g/h an. Der Kopf der Waschkolonne 10 wird stündlich über die Leitung 20 mit 3 l Wasser von 25 °C beaufschlagt.

**Ansprüche**

1. Verfahren zur Herstellung von $C_2$- bis $C_4$-Olefinen aus Methanol, Dimethylether und gegebenenfalls Wasserdampf enthaltenden Gasgemischen in Gegenwart von Katalysatoren bei Temperaturen von 250 bis 500 °C und Drucken von 0,1-6 bar unter Rückführung der nicht umgesetzten Ausgangsstoffe, wobei man zu Beginn Methanol oder ein Methanol/Wasser-Gemisch im Volumenverhältnis 1 : (0,1 bis 2) verdampft und in einem den Katalysator enthaltenden Reaktor umsetzt, die Reaktionsgase durch Abkühlung teilkondensiert, in einem Abscheider in drei Phasen auftrennt und die aus höheren Aliphaten und Aromaten bestehende Ölphase abzieht, dadurch gekennzeichnet, daß man die verbleibende wäßrige Phase und die Gasphase in einer Waschkolonne bei Drucken von 1-40 bar mit Wasser wäscht, über Kopf ein überwiegend $C_2$-bis $C_4$-Olefine enthaltendes gasförmiges Kohlenwasserstoffgemisch abzieht und in an sich bekannter Weise auftrennt, aus der Blase der Waschkolonne eine das nicht-umgesetzte Methanol und den als Zwischenprodukt gebildeten Dimethylether enthaltende wäßrige Phase abzieht und daraus in einer Abstreifkolonne Methanol und Dimethylether abdestilliert und in den Reaktor zurückführt, während man aus der Blase der Abstreifkolonne das Wasser abzieht.

2. Verfahren nach Anspruch 1, dadurch gekennzeichnet, daß der Katalysator einen Zeolith vom Typ H ZSM-5 enthält und die Umsetzung bei Drucken von 0,1-3 bar und, entsprechend dem allmählichen Aktivitätsabfall des Katalysators, bei von 280° bis 360 °C ansteigenden Temperaturen stattfindet.

3. Verfahren nach Anspruch 1 oder 2, dadurch gekennzeichnet, daß man durch Steuerung der Reaktionstemperatur das Methanol bei jedem Durchgang jeweils nur bis zu höchstens 80 % zu Kohlenwasserstoffen umsetzt.

4. Verfahren nach einem der Ansprüche 1-3, dadurch gekennzeichnet, daß man in der Waschkolonne 1 Liter Waschwasser je 3-200 Normliter über Kopf abgezogenes, gasförmiges Kohlenwasserstoffgemisch einsetzt.

5. Verfahren nach einem der Ansprüche 1-4, dadurch gekennzeichnet, daß die Temperatur in der Blase um 5-40 °C höher liegt als am Kopf der Waschkolonne.

6. Verfahren nach einem der Ansprüche 1-5, dadurch gekennzeichnet, daß man einen Teil der aus der Blase der Waschkolonne abgezogenen Waschlösung verdampft und in den Reaktor zurückführt.

7. Verfahren nach einem der Ansprüche 1-6, dadurch gekennzeichnet, daß die Abstreifkolonne bei der Siedetemperatur des Wassers betrieben wird.

8. Verfahren nach einem der Ansprüche 1-7, dadurch gekennzeichnet, daß man von dem aus der Blase der Abstreifkolonne abgezogenen Wasser mindestens einen dem jeweils gebildeten Reaktionswasser entsprechenden Anteil aus dem System ausschleust.

9. Verfahren nach einem der Ansprüche 1-8, dadurch gekennzeichnet, daß man einen Teil des aus der Blase der Abstreifkolonne abgezogenen Wassers abkühlt und auf den Kopf der Waschkolonne zurückführt.

10. Verfahren nach einem der Ansprüche 1-9, dadurch gekennzeichnet, daß man einen Teil des aus der Blase der Abstreifkolonne abgezogenen Wassers zur Verdünnung des Methanols in den Reaktor zurückführt.

## Claims

1. Process for making $C_2$ to $C_4$-olefins from gas mixtures containing methanol, dimethylether and optionally steam in the presence of catalysts at temperatures of 250 to 500 °C under pressures of 0.1 to 6 bars, unreacted feed material being recycled, wherein methanol or methanol/water-mixture in a ratio by volume of 1 : (0.1 to 2) is initially evaporated and reacted in a reactor having the catalyst placed therein, the reaction gases are partially condensed by cooling them and separated, in a separator, into three phases, and the oil phase consisting of higher aliphatic and aromatic hydrocarbons is removed, which comprises : water-scrubbing the remaining aqueous phase and gas phase in a scrubbing column under pressures of 1 to 40 bars ; removing overhead a gaseous hydrocarbon mixture containing prodominantly $C_2$ to $C_4$-olefins and separating it into its components in known manner ; removing, from the base portion of the scrubbing column, and aqueous phase containing unreacted methanol and intermediarily formed dimethylether ; distilling off the methanol and dimethylether in a stripping column and recycling them into the reactor ; and removing the water from the base portion of the stripping column.

2. Process as claimed in claim 1, wherein the catalyst contains a H ZSM-5 type zeolite and the reaction takes place under pressures of 0.1 to 3 bars and at temperatures increasing from 280 to 360 °C, consistently with the gradually decreasing catalyst activity.

3. Process as claimed in claim 1 or 2, wherein the methanol is permitted to undergo conversion to hydrocarbons to a maximum extent of only 80 %, during each passage, by controlling the temperature.

4. Process as claimed in any of claims 1 to 3, wherein 1 liter scrubbing water is used in the scrubbing column per 3 to 200 normal liters gaseous hydrocarbon mixture removed overhead.

5. Process as claimed in any of claims 1 to 4, wherein the temperature in the base is by 5 to 40 °C higher than the temperature in the head of the scrubbing column.

6. Process as claimed in any of claims 1 to 5, wherein a portion of the scrubbing solution removed from the base of the scrubbing column is evaporated and recycled into the reactor.

7. Process as claimed in any of claims 1 to 6, wherein the stripping column is operated at the boiling temperature of water.

8. Process as claimed in any of claims 1 to 7, wherein a portion of the water taken from the base of the stripping column is removed from the system, said portion corresponding at least to the reaction water formed during each passage.

9. Process as claimed in any of claims 1 to 8, wherein a portion of the water removed from the base of the stripping column is cooled and recycled to the head of the scrubbing column.

10. Process as claimed in any of claims 1 to 9, wherein a portion of the water removed from the base of the stripping column is recycled to the reactor for diluting the methanol therein.

## Revendications

1. Procédé de préparation d'oléfines en $C_2$-$C_4$ à partir de méthanol, d'éther diméthylique et de mélanges gazeux contenant, éventuellement, de la vapeur d'eau en présence de catalyseurs, à des températures de 250 à 500 °C et sous des pressions de 0,1 à 6 bars, avec recyclage des matières de départ n'ayant pas réagi, dans lequel on évapore au début du méthanol ou un mélange méthanol/eau dans un rapport en volume de 1 : (0,1-2) et on le fait réagir dans un réacteur contenant le catalyseur, on condense partiellement les gaz de réaction par refroidissement, on les sépare dans un séparateur en trois phases et on élimine la phase huileuse consistant en hydrocarbures aliphatiques et aromatiques supérieurs, caractérisé en ce qu'on lave à l'eau dans une colonne de lavage la phase aqueuse restante et la phase gazeuse sous une pression de 1-40 bars, on élimine en tête un mélange d'hydrocarbures gazeux contenant une quantité prépondérante d'oléfines en $C_2$-$C_4$ et on le sépare en ses constituants de manière connue, on élimine du fond de la colonne de lavage une phase aqueuse contenant le méthanol n'ayant pas réagi et l'éther diméthylique formé comme produit intermédiaire et on en élimine le méthanol et l'éther diméthylique par distillation dans une colonne d'entraînement et on les recycle dans le réacteur, tandis qu'on soutire l'eau du fond de la colonne d'entraînement.

2. Procédé selon la revendication 1, caractérisé en ce que le catalyseur renferme une zéolite du type H ZSM-5 et la réaction se déroule sous des pressions de 0,1 à 3 bars et à des températures augmentant de 280 à 360 °C en fonction de la diminution progressive d'activité du catalyseur.

3. Procédé selon la revendication 1 ou 2, caractérisé en ce que, par réglage de la température de réaction, on ne convertit à chaque passage que 80 % au maximum du méthanol en hydrocarbures.

4. Procédé selon l'une des revendications 1 à 3, caractérisé en ce que l'on utilise dans la colonne de lavage 1 litre d'eau de lavage pour 3 200 litres normaux de mélange d'hydrocarbures gazeux éliminé en tête.

5. Procédé selon l'une des revendications 1 à 4, caractérisé en ce que la température dans le fond est supérieure de 5-40 °C à celle en tête de la colonne de lavage.

6. Procédé selon l'une des revendications 1 à 5, caractérisé en ce que l'on évapore une partie de la solution de lavage soutirée du fond de la colonne de lavage et on la recycle dans le réacteur.

7. Procédé selon l'une des revendications 1 à 6, caractérisé en ce que l'on exploite la zone d'entraînement à la température d'ébullition d'eau.

8. Procédé selon l'une des revendications 1 à 7, caractérisé en ce que l'on retire du système au moins une quantité de l'eau éliminée du fond de la colonne d'entraînement correspondant à la quantité d'eau de réaction formée chaque fois.

9. Procédé selon l'une des revendications 1 à 8, caractérisé en ce que l'on refroidit une partie de l'eau soutirée du fond de la colonne d'entraînement et on la recycle dans le réacteur.

10. Procédé selon l'une des revendications 1 à 9, caractérisé en ce qu'une partie de l'eau soutirée du fond de la colonne d'entraînement est recyclée dans le réacteur pour diluer le méthanol.

CH$_3$OH  H$_2$O

0 065 112